# EUROPÄISCHE PATENTSCHRIFT

(11) **EP 2 566 419 B1**
(45) Veröffentlichungstag und Bekanntmachung des Hinweises auf die Patenterteilung: **09.01.2019**
(21) Anmeldenummer: 11718991.0
(22) Anmeldetag: 06.05.2011
(51) Int. Cl.: A61F 5/01

(54) **ORTHESE**
ORTHOSIS
ORTHÈSE

(30) Priorität: 07.05.2010 DE 102010019845
(43) Veröffentlichungstag der Anmeldung: 13.03.2013
(73) Patentinhaber: Gottinger Handelshaus OHG, 85604 Zorneding (DE)
(72) Erfinder: GÜNTHER, Norbert, G., 85599 Parsdorf (DE)
(74) Vertreter: Winter, Brandl, Fürniss, Hübner, Röss, Kaiser, Polte - Partnerschaft mbB
(86) Internationale Anmeldenummer: PCT/EP2011/057356
(87) Internationale Veröffentlichungsnummer: WO 2011/138453

(56) Entgegenhaltungen:
- EP-A1- 2 272 471
- EP-A1- 2 272 471
- WO-A1-00/74946
- WO-A1-2006/076932
- WO-A2-02/065942
- WO-A2-2008/066856
- DE-U1-202004 012 892
- US-A1- 2005 277 859
- US-A1- 2006 135 901
- MARIO C FAUSTINI ET AL: "Manufacture of Passive Dynamic Ankle-Foot Orthoses Using Selective Laser Sintering", IEEE TRANSACTIONS ON BIOMEDICAL ENGINEERING, IEEE SERVICE CENTER, PISCATAWAY, NJ, USA, Bd. 55, Nr. 2, 1. Februar 2008 (2008-02-01), Seiten 784-790, XP011200272, ISSN: 0018-9294, DOI: 10.1109/TBME.2007.912638

## Beschreibung

Die vorliegende Erfindung betrifft ein Verfahren zur Herstellung einer Orthese zumindest bestehend aus einem schalen- oder schellenformigen Oberteil, einem schalen- oder schellenförmigen Unterteil sowie einem das Ober- und Unterteil koppelnden Zwischenelement gemäß dem Oberbegriff des Patentanspruchs 1 sowie eine nach einem derartigen Verfahren hergestellte Orthese.

Als Orthese wird in der Regel ein medizinisches Hilfsmittel bezeichnet, das zur Unterstützung von eingeschränkt funktionsfähigen Körperteilen zum Einsatz gebracht wird. Hierfür existieren Orthesen zur Fixierung von Körperteilen, zur Korrektur von Körperteilfehlstellungen, zum Ausgleich von Längendifferenzen zwischen Körperteilen oder zur Bewegungsstabilisierung und/oder Ruhigstellung von Gelenken. Sie werden grundsätzlich äußerlich an das zu therapierende Körperteil angelegt und über längere Zeiträume getragen. Um hierbei den Tragekomfort zu erhöhen, d.h. insbesondere Druckstellen zu vermeiden und trotzdem einen stabilen, engen Sitz am betreffenden Körperteil zu erreichen, sind Orthesen moderner Bauart den ergonomischen Verhältnissen angepasst oder weitestgehend angenähert. Hierfür werden neuartige Werkstoffe wie beispielsweise Verbundwerkstoffe (Kohlefaser- / Glasfaserverstärkte Kunststoffe), Aluminiumlegierungen, etc. verwendet, die sich der äußeren Form des zu therapierenden Körperteils angleichen lassen und so großflächig an das betreffende Körperteil anlegbar sind. Hierdurch wird nicht nur der Sitz der Orthese optimiert sondern es wird auch bewirkt, dass äußere Kräfte großflächig an die Oberfläche des zu therapierenden Körperteils abgegeben werden können und somit keine Druckstellen erzeugen.

Aus dem Stand der Technik, beispielsweise gemäß der DE 10 2004 027 252 A1 als eine mögliche Ausgestaltung einer Orthese, ist eine sogenannte Fußgelenkorthese bekannt, bestehend aus einem Fußteil und einem Unterschenkelteil, die über eine vorgekrümmte Blattfeder als koppelndes Zwischenelement miteinander biegeelastisch verbunden sind. Das Unterschenkel- und Fußteil besteht jeweils aus einer Kunststoffschale, die längsgeschlitzt sind, sodass die Orthese um den Unterschenkel sowie den Fuß gelegt werden kann. Ferner sind an den Kunststoffschalen jeweils Schnallen oder mit Klettverschlüssen versehene Bänder vorgesehen, mittels denen die Längsschlitze zusammengezogen werden können, um die Unterschenkel- und Fußschale eng an den Unterschenkel und den Fuß des Patienten anzulegen. Schließlich weisen Unterschenkel- und Fußschalen äußere Aufnahmeschächte auf, in welche das Federelement, vorliegend in Form der Blattfeder, eingesteckt ist.

Derartige Orthesen gibt es für nahezu sämtliche Gliedmaßen eines Patienten, wobei je nach Aufgabe und Therapiesituation anstelle des vorstehend beschriebenen Federelements ein starres Gestänge oder ein Scharnier als das Ober- und Unterteil koppelnde Zwischenelement vorgesehen sein kann.

Es hat sich jedoch herausgestellt, dass trotz ergonomischer Formgebung insbesondere von Ober- und Unterteil einer gattungsgemäßen Orthese der Tragekomfort vorzugsweise bei längerer Tragedauer nicht optimal ist. Entscheidend hierbei ist das Scheuerverhalten der Orthese auf der Haut des Patientenkörperteils. Polsterungen und Ausschäumungen eignen sich zwar dazu, Druckstellen zu vermeiden und einen gewissen Flüssigkeitstransport zu gestatten. Sie haben jedoch den Nachteil, dass sich der Sitz der Orthese verschlechtert (Lageänderung der Orthese infolge nachgiebiger Polsterungen) und dass die Krafteinleitung in das Körperteil ungenauer wird. Die Wirkung der Orthese verschlechtert sich dementsprechend. Auch bilden Polster ein Ort von Bakterienansammlungen mit entsprechenden Auswirkungen auf Aussehen, Gerüchen und ggf. sogar allergischen Reaktionen seitens des Patienten. Letztlich sind Polsterungen weniger langlebig als das übrige Orthesenmaterial und verschleißen daher schnell. Hierdurch leidet das äußere Erscheinungsbild der Orthese erheblich.

Unabhängig von der vorstehend beschriebenen Problematik bei bekannten Orthesen ist die Trageakzeptanz insbesondere bei Kindern und Jugendlichen nicht besonders ausgeprägt. Damit wird der orthopädische Therapieerfolg häufig in Frage gestellt. Dies liegt zum Einen am mangelnden Tragekomfort, wie dies vorstehend beschrieben wurde und zum Anderen an der Tatsache einer medizinisch notwendigen Bewegungseinschränkung bei gleichzeitiger Einbuße des optischen Erscheinungsbilds des Patienten. In anderen Worten ausgedrückt, überwiegen bei der Konstruktion einer Orthese funktionelle Notwendigkeiten und weniger die optische Attraktivität. Letzteres wird durch besagte Aufpolsterungen vollständig ruiniert.
Zur Verringerung des fertigungstechnischen Aufwandes wird in den Druckschriften WO 2008/066856 A2 und Mario C. Faustini et al, Manufacture of Passive Dynamic Ankle-Foot Orthoses Using Selective Laser Sintering "IEEE TRANSACTIONS ON BIOMEDICAL ENGINEERING, Vol. 55, NO. 2, February 2008, die den nächstliegenden Stand der Technik darstellen, vorgeschlagen, eine Orthese nach dem "Rapid Prototyping"-Verfahren herzustellen.
Der Grundaufbau von Knie-Orthesen ist aus den Druckschriften US 2006/0135901 A1, US 2005/0277859 A1 und WO 00/74946 A1 bekannt.
In den Druckschriften WO 2006/076932 A1 und DE 20 2004 012 892 U1 wird vorgeschlagen, zur Belüftung die Orthese mit Belüftungsöffnungen auszuführen.
Der Erfindung liegt die Aufgabe zu Grunde, ein Verfahren zum Herstellen einer Orthese und einen nach einem derartigen Verfahren hergestellte Orthese bereitzustellen, bei der der fertigungstechnische Aufwand verringert ist.
Es ist weiterhin Aufgabe der vorliegenden Erfindung, ein Verfahren zur Herstellung einer Orthese bereit zu stellen, die bei zumindest gleichbleibender Funktionalität eine gesteigerter Trageakzeptanz bei Patienten bewirkt und damit einen höheren therapeutischen Effekt erzielt. Ein Ziel ist es, die Funktionalität der Orthese durch eine erhöhte Flexibilität bezüglich deren Anwendungsmöglichkeiten zu verbessern.
Diese Aufgabe wird durch ein Verfahren zur Herstellung einer Orthese mit den Merkmalen des Patentanspruchs 1 beziehungsweise eine nach einem derartigen Verfahren hergestellte Orthese gelöst.
Vorteilhafte Ausgestaltungen der Erfindung sind Gegenstand der Unteransprüche.

Grundsätzlich orientiert sich die Form einer Orthese nach dem Körperteil eines Patienten, an den sie angelegt werden soll. Diese technische Ausgestaltung ergibt sich letztlich aus funktionellen Anforderungen an die Orthese bezüglich deren orthopädische Wirkung sowie deren Tragekomfort. Trotz dieser Notwendigkeiten erlaubt es der Einsatz moderner Materialien und Herstellungsverfahren, ohne großen Kostenaufwand sowie ohne Reduzierung der funktionellen Eigenschaften insbesondere jene Bestandteile der Orthese, die schalenförmig großflächig mit den zu therapierenden Körperteilen in Kontakt kommen, als stilistische Formenträger vorzusehen. D.h. insbesondere die ergonomisch oberflächenangepassten/-angenäherten Bauteile einer Orthese können erfindungsgemäß dazu genutzt werden, die körperteilbedingte Form der Orthese zu "verschleiern" oder zu "überlagern" und damit für Patienten optisch attraktiver zu machen.

Gemäß der Erfindung ist es vorgesehen, zumindest das Ober- und/oder Unterteil einer Orthese nach dem "Rapid Prototyping" bzw. dem "Rapid Manufacturing" - Verfahren herzustellen, wonach nach dem Prinzip des generativen Fertigungsverfahrens geometrische Werte am zu therapierenden Körperteil (z.B. per Lasertechnologie) vorzugsweise 3-dimensional abgegriffen und als CAD-Daten abgespeichert werden. Diese CAD-Daten bezüglich der individuellen Körperteilgeometrie werden dann direkt in ein entsprechendes Werkstück, nämlich zumindest das Ober- und/oder Unterteil der Orthese umgesetzt. Hierfür wird das Werkstück in an sich bekannter Weise schichtweise aus formlosem oder formneutralem Material (beispielsweise Sintermaterial) unter Ausnutzung physikalischer und/oder chemischer Effekte aufgebaut. Durch diese Herstellungsweise kann die Orthese wesentlich genauer an die individuelle Ergonomie des zu therapierenden Körperteils angepasst und damit der Tragekomfort verbessert werden. Die Notwendigkeit der Aufpolsterung der Orthese reduziert sich damit, da keine Druckpunkte infolge Passungenauigkeiten ausgeglichen werden müssen. Bereits aus diesem Grund wird die Trageakzeptanz bei einem Patienten angehoben.

Gemäß der Erfindung können zumindest das Ober- und/oder Unterteil der Orthese in Abhängigkeit des zu therapierenden Körperteils wenigstens äußerlich einem anderen Bauteil/Körperteil gleicher oder ähnlicher Funktion wie das zu therapierende Körperteil angenähert werden. So kann es beispielsweise vorgesehen sein, mit der erfindungsgemäßen Orthese ein Maschinenelement oder ein entsprechendes Körperteil eines anderen Lebewesens durch entsprechende äußere Formgebung zumindest des (schalenförmigen) Ober- und/oder Unterteils zu simulieren, um die Orthese an das individuelle Geschmacksempfinden des Patienten anzupassen. Hierdurch wird die durchschnittliche Trageakzeptanz gesteigert und die orthopädische Wirkung der "gestilten" Orthese indirekt verbessert. Hierfür wird das vorstehend genannte "Rapid Prototyping"-Verfahren eingesetzt, wobei den eingelesenen Ergonomiedaten bezüglich des individuellen Patientenkörperteils vorab abgespeicherte standardisierte Formdaten bezüglich des simulierten Körper- oder Maschinenteils überlagert werden. Im Ergebnis wird hierdurch eine Form erhalten, die äußerlich dem simulierten Körperteil/Maschinenteil angenähert ist, jedoch nach wie vor exakt der Ergonomie des zu therapierenden Körperteils des Patienten entspricht und damit höchsten Tragekomfort gewährleistet.

Gemäß einer Weiterbildung sieht die Erfindung prinzipiell ein Belüftungssystem der Orthese vor. Dieser Überlegung liegt die Erkenntnis zugrunde, dass die Bildung/Ansammlung von Feuchtigkeit auf der Hautoberfläche im Bereich der Orthese die Entstehung von Scheuerstellen an der Haut nachhaltig begünstigt. Auch bildet Feuchtigkeit ein Nährboden für Bakterien und Pilze, die den Zustand der Haut im Bereich der Orthese weiter verschlechtern. Die Ausbildung eines solchen Belüftungssystems reduziert oder verhindert einen Feuchtigkeitsstau im Orthesenbereich und erhöht so den Tragekomfort. Das erfindungsgemäße Belüftungssystem eröffnet darüber hinaus die Möglichkeit, die Oberfläche der Orthese und insbesondere des Ober- und Unterteils unter Beibehaltung oder Erzeugung der Belüftungswirkung so zu verändern, dass ein ästhetisch verbesserter Gesamteindruck entsteht. In anderen Worten ausgedrückt, kann das Belüftungssystem selbst eine designerische / ästhetische Aufwertung der Orthese bewirken und/oder erbringt den Vorteil einer weiter reduzierten notwendigen Aufpolsterung, sodass hierdurch Freiräume geschaffen werden, die nunmehr zu stilistischen Zwecken nutzbar sind.

Im Konkreten hat eine erfindungsgemäße Orthese nach dieser Weiterbildung der Erfindung zumindest ein schalen- oder schellenförmiges Oberteil, ein schalen- oder schellenförmiges Unterteil sowie ein das Ober- und Unterteil koppelndes Zwischenelement. Zumindest eines aus dem Ober- und Unterteil besteht aus einer an die äußere Kontur des betreffenden Körperteils formtechnisch angepassten oder angenäherten Schale vorzugsweise aus Kunststoff, die wiederum weiter vorzugsweise aus mehreren Schalenteilen (Schalenhälften) zusammengesetzt ist, die beispielsweise mittels Spannelemente für einen festen Körpersitz verspannbar sind. Wenigstens eine der mehreren verspannbaren Schalenteile weist eine Perforation beispielsweise in Form von Durchgangslöchern, oder Spalte auf, die eine Transpiration durch das Schalenteil hindurch erlauben.

Die Perforation kann dabei den optischen Eindruck eines künstlerischen Motivs ergeben. Auch besteht die Möglichkeit, die Perforationsöffnungen durch in das Schalenteil eingeprägte, eingravierte oder anderweitig aufgedruckte Linien zu dem künstlerischen Motiv zu verbinden.

Schließlich ergibt sich die grundsätzliche Möglichkeit sowohl im Perforationsbereich also auch in hierzu parallelen Bereichen der Orthese unabhängig von der Anordnung der Perforationsöffnungen künstlerische Motive anzubringen, d.h. die Orthese selbst als Gestaltungsfläche für deren stilistisches Aufwerten zu nutzen, wie dies vorstehend bereits ausgeführt wurde. Begünstigt wird dies prinzipiell dadurch, dass gegenüber dem Stand der Technik weniger Polstermaterial erforderlich ist, um beispielsweise eine Transpiration zu ermöglichen und damit die gestalterischen Möglichkeiten ohne Einbuße der Funktionalität der Orthese vergrößert werden. Auf diese Weise können Funktion und Design nahezu kompromisslos miteinander verknüpft werden.

Es hat sich als besonders vorteilhaft erwiesen, wenn die Perforation ebenfalls als standardisierte Formendaten im CAD-System abgespeichert ist und den eingelesenen Ergonomiedaten des Patientenkörperteils überlagert wird.

Vorzugsweise haben die Perforationslöcher an der dem zu therapierenden Körperteil zugewandte Schaleninnenseite einen kleineren Durchmesser als an der Schalenaußenseite, um die Körperteiloberfläche vor Druckstellen (sogenannte Fensterödeme zu schützen.

Die Orthese kann mit drei längs beabstandeten Schalen ausgebildet werden, bestehend aus
einer Oberschenkelschale, einer Unterschenkelschale und einer Fußschale. Ober- und Unterschenkelschale sind über ein Scharnier mit einander gekoppelt, dessen zusammenwirkende Scharnierelemente einstückig mit der Ober- und Unterschenkelschale hergestellt sind. Unterschenkel- und Fußschale sind vorzugsweise über ein externes Verbindungselement, insbesondere eine Blattfeder miteinander verbunden, die in entsprechende Aufnahmen an der Unterschenkel- und Fußschale eingesteckt ist, welche ebenfalls einstückig mit der entsprechenden Schale ausgeformt sind.

Schließlich sind die Schalen der Orthese vorzugsweise mit (integralen) Versteifungsrippen ausgeformt, die insbesondere die Anschlussbereiche der Schalen, d.h. vorliegend die Scharnierelemente sowie die Aufnahmen für die Blattfeder mit einschließen, um die Schalen besonders in diesen belasteten Bereichen (Krafteinleitpunkte) zu verstärken. Die Rippen können dabei bevorzugt den Spannungsverläufen innerhalb der jeweiligen Schale nachgezogen sein, wie sie sich bei der Verwendung der Orthese ergeben.

Die Erfindung wird nachstehend anhand bevorzugter Ausführungsbeispiele unter Bezugnahme auf die begleitenden Figuren näher erläutert.
Fig. 1a bis 1e zeigt ein Studie prinzipieller Designs vorliegend für eine kombinierte Fuß- und Kniegelenkorthese bestehend aus drei Körperschalen mit zwischengefügtem (Knie-) Gelenkscharnier und (Knöchel-Versteifungsfeder als mögliche Beispiele für eine technischer Umsetzung des erfindungsgemäßen Konzepts.
Fig. 2 zeigt eine Schafthälfte eines Unterschenkelteils einer Fußgelenkorthese mit Belüftungsöffnungen gemäß einem Ausführungsbeispiel der Erfindung,
Fig. 3 und 4 zeigen die Schafthälfte der Fig. 2 in vergrößerten Darstellungen,
Fig. 5a und 5b zeigt die kombinierte Fuß- und Kniegelenkorthese gemäß einem weiteren Ausführungsbeispiel der Erfindung, hergestellt nach dem "Rapid-Prototyping"-Verfahren (gemäß der Fig. 1),
Fig. 6 zeigt eine Gegenüberstellung von Orthesenformen gemäß dem Stand der Technik und Designstudien für Orthesen, die nach dem erfindungsgemäßen Fertigungskonzept hergestellt sind,
Fig. 7, 8 zeigen ein anderes Ausführungsbeispiel zur "Verschleierung" von ergonomisch bedingten Formen für Orthesenschalen durch Oberflächenbearbeitung,
Fig. 9 - 11 zeigen ein Ausführungsbeispiel zur "Verschleierung" von ergonomisch bedingten Formen für Orthesenschalen durch Überlagerung erfasster Körperteil-Formendaten mit vorprogrammierten geometrischen Standarddaten,
Fig. 12 zeigt eine Perspektivenansicht der kombinierten Fuß- und Kniegelenkorthese gemäß der Erfindung,
Fig. 13 zeigt eine Seitenansicht der kombinierten Fuß- und Kniegelenkorthese gemäß der Erfindung,
Fig. 14 zeigt eine Schnittansicht durch das Kniegelenkscharnier der erfindungsgemäßen Orthese,
Fig. 15 zeigt eine Perspektivenansicht der Fußschale der kombinierten Fuß- und Kniegelenkorthese gemäß der Erfindung,
Fig. 16 zeigt eine Vorderansicht der Unterschenkelschale der kombinierten Fuß- und Kniegelenkorthese gemäß der Erfindung und
Fig. 17 zeigt eine Unteransicht der Unterschenkelschale der kombinierten Fuß- und Kniegelenkorthese gemäß der Erfindung.

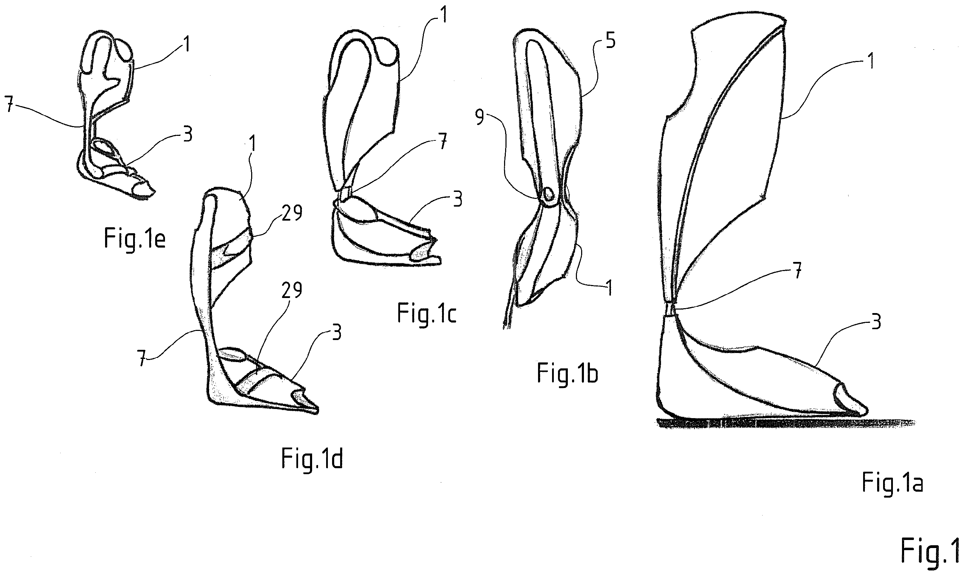

In Fig. 1 ist eine allgemeine Designstudie für Fuß- und/oder Kniegelenk-Orthesen unterschiedlicher Anwendungsbereiche dargestellt, um die Gestaltungsmöglichkeiten bei Anwendungen des sogenannten "Rapid-Prototyping"-Verfahrens gemäß der Erfindung zu dokumentieren.
Wie vorstehend bereits angedeutet wurde, hat eine Orthese wenigstens zwei Krafteinleitungselemente nämlich ein Ober- und ein Unterteil 3, 5 sowie ggf. einem Mittelteil 1, die mit einem Teil eines Patientenkörpers unmittelbar in Kontakt kommen. In der Regel dient die Orthese dazu, ein Körpergelenk zu brücken, indem beispielsweise eine Versteifungsschiene 7 zur Ruhigstellung des betreffenden Gelenks oder ein Scharnier 9 zur geführten/eingeschränkten Bewegung des betreffenden Gelenks vorgesehen ist, das als Kopplungselement zwischen dem Orthesenober-, -mittel- und -unterteil 1-5 eingefügt ist. Wie ferner bereits vorstehend ausgeführt wurde, sind zumindest das Ober-, ggf. Mittel- und Unterteil 1-5 der Orthese an die äußere Form des betreffenden Körperteils angepasst, um möglichst flächig an dem Körperteil anzuliegen und somit Krafteinleitungsspitzen und damit verbundene Druckstellen zu vermeiden. Hierdurch ist es möglich, Aufpolsterungen deutlich zu reduzieren, sodass der Orthesensitz insgesamt verbessert wird. Erfindungsgemäß ist es nunmehr vorgesehen, nach dem Prinzip des "Rapid-Prototyping"-Verfahrens die äußere Geometrie des betreffenden Körperteils des Patienten individuell zu erfassen und in Form von (3-dimensionalen) CAD-Daten digital abzuspeichern. In einem nächsten Schritt werden dann zumindest das Ober-, ggf. Mittel- und Unterteil 1-5 entsprechend den eingespeicherten CAD-Daten direkt aus geeignetem Material, beispielsweise Sintermaterial schichtweise aufgebaut. Damit wird erreicht, dass zumindest das Ober- und Unterteil 1-5 der Orthese exakt dem betreffenden Körperteil des Patenten entspricht und damit ein perfekter Sitz garantiert ist. Es liegt dabei auf der Hand, dass durch die exakte Anpassung der Orthese an den betreffenden Körperteil des Patienten die Notwendigkeit einer Aufpolsterung zum Ausgleich von Passungenauigkeiten prinzipiell entfällt, sodass Polster nur sehr sparsam eingesetzt werden können. Damit bleibt die Formgebung der Orthese insbesondere des Ober-, Mittel- und/oder Unterteils im Wesentlichen sichtbar erhalten. Dieser Umstand bietet nunmehr eine Vielzahl von Möglichkeiten zur Dimensionierung der Orthese, wie diese auszugsweise in der Fig. 1 angedeutet sind.

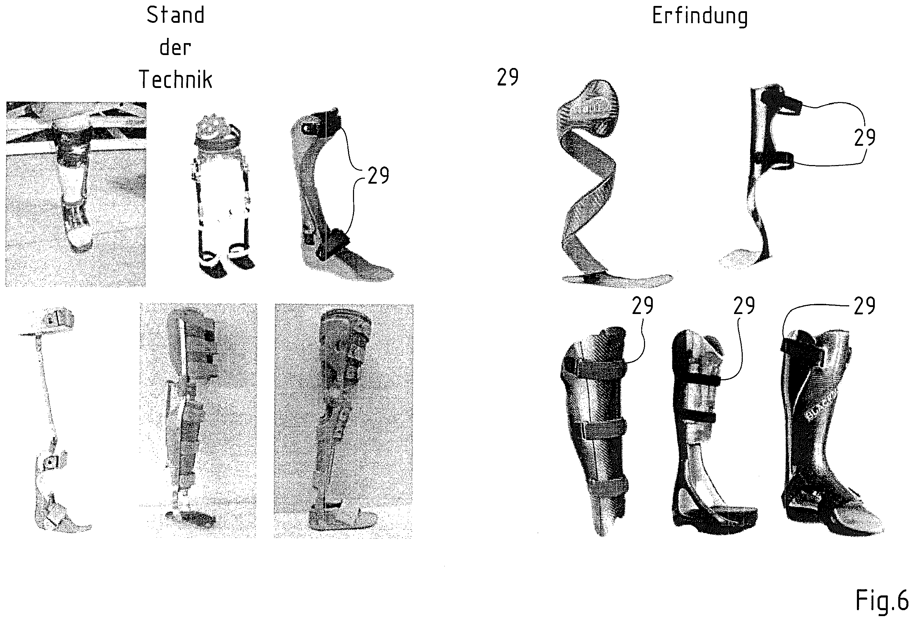

In der Fig. 6 sind unterschiedliche Varianten für eine Fußgelenkorthese und eine Kniegelenkorthese ggf. in Kombination angegeben. Diese Orthesen unterscheiden sich offenkundig durch ihr sichtbares äußeres Erscheinungsbild, gleichen sich jedoch bezüglich ihrer inneren Abmessungen entsprechend den tatsächlichen Abmessungen des zu therapierenden Körperteils des Patienten. D.h. die Anwendung des "Rapid-Prototyping"-Verfahrens erlaubt es, der natürlich vorgegebenen Form und Abmessung individuell bezogen (patientenbezogen) auf das zu therapierende Körperteil eine "künstliche Form" überzulegen (zu überlagern), um trotz Beibehaltung der natürlich vorgegebenen (individuellen) Form eine hievon abweichende Gestalt zu simulieren. So ist es beispielsweise möglich, das die Ober-, ggf. Mittel- und Unterteile 1-5 miteinander jeweils koppelnde Zwischenelement 7, 9 als ein externes Bauteil vorzusehen, das an speziell gestalteten Anlenkpunkten am Ober-, Mittel- und Unterteil montiert ist oder in das Ober-, Mittel- und Unterteil zu integrieren (einstückige Ausbildung). Je nach Ausführung ergibt sich ein völlig unterschiedlicher Gesamteindruck bei gleicher Funktionalität. Insbesondere ist es bei einem Scharniermechanismus 9 als koppelndes Zwischenelement vorteilhaft, die einzelnen Scharnierelemente einstückig mit dem Ober- und Unterteil 3, 5 auszuformen, um in diesem Fall eine futuristische Komposition zu erhalten. Auch besteht die Möglichkeit, das Ober-, Mittel- und/oder Unterteil 1-5 aus mehreren Teilschalen zu bilden, die bei Anwendung des "Rapid-Prototyping"-Verfahrens passgenau aneinander fügbar sind. Damit wird nicht nur das An- und Ablegen der Orthese erleichtert sondern es bieten sich unterschiedliche Gestaltungsmöglichkeiten für die einzelnen Schalenteile an.

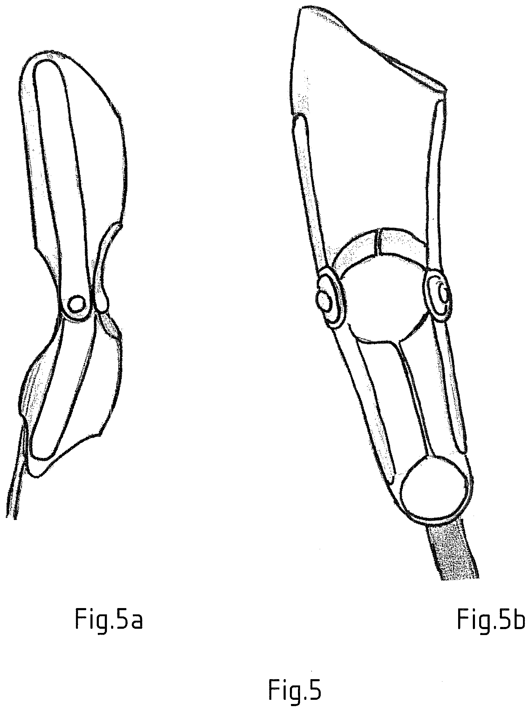

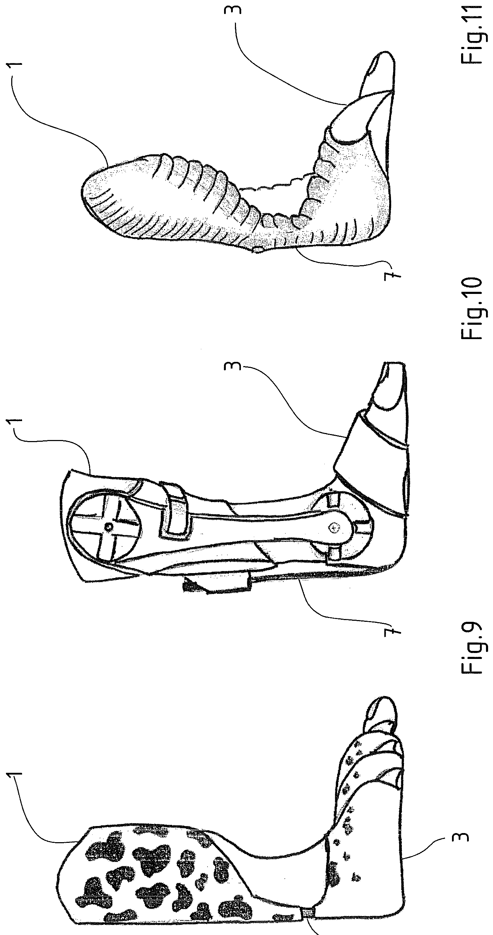

In den Fig. 5, 6 sowie 9 bis 11 sind weitere Ausgestaltungen für eine Orthese, vorliegend in Form einer Fußgelenkorthese als ein Beispiel dargestellt, um die Vielfalt gestalterischer Möglichkeiten bei der Anwendung des "Rapid-Prototyping"-Verfahrens zu zeigen.

Sämtliche dargestellten Fußgelenkorthesen entsprechen einem und demselben natürlichen Körperteil eines einzigen Patienten, das nach dem genannten Verfahren vermessen und die hieraus gewonnenen CAD-Daten digital abgespeichert wurden. Parallel hierzu enthält der Speicher (standartisierte) CAD-Daten von künstlichen Gestaltungen wie beispielsweise entsprechende Körperteile von Tieren oder Maschinen. Hier ist der Phantasie eines Designers keine Grenzen gesetzt.

Erfindungsgemäß werden nunmehr die individuellen Messdaten mit den Standard-Daten kombiniert, d.h. die Messdaten werden von den Standarddaten überlagert, sodass eine äußerlich der künstlichen Gestaltung entsprechende Orthesenform erhalten wird, deren Abmessungen und (innere) Form jedoch nach wie vordem individuellen Körperteil des Patienten entsprechen. So ist es möglich, der Orthese im beispielhaften Fall einer Fußgelenkorthese beispielsweise die äußere Form eines Tierfußes oder eines Roboters zu geben. Auch filigrane Phantasieformen jeglicher Art sind auf diese Weise verwirklichbar.

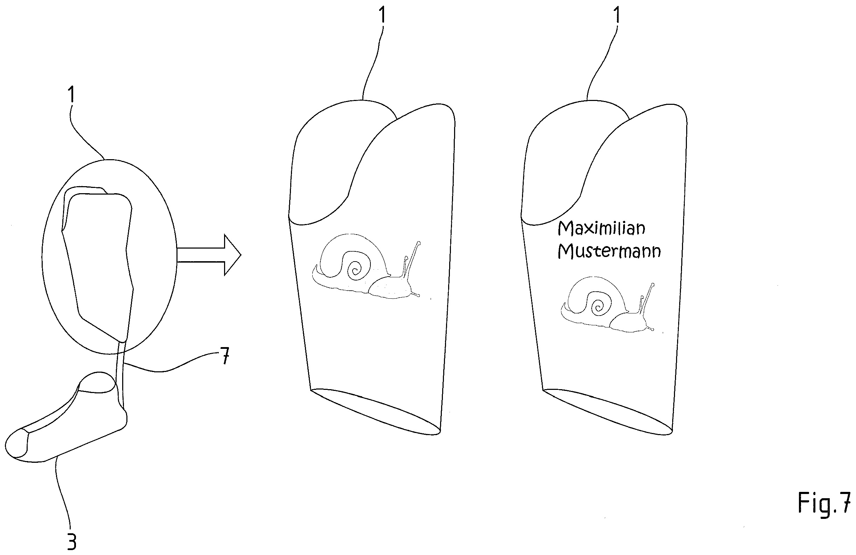

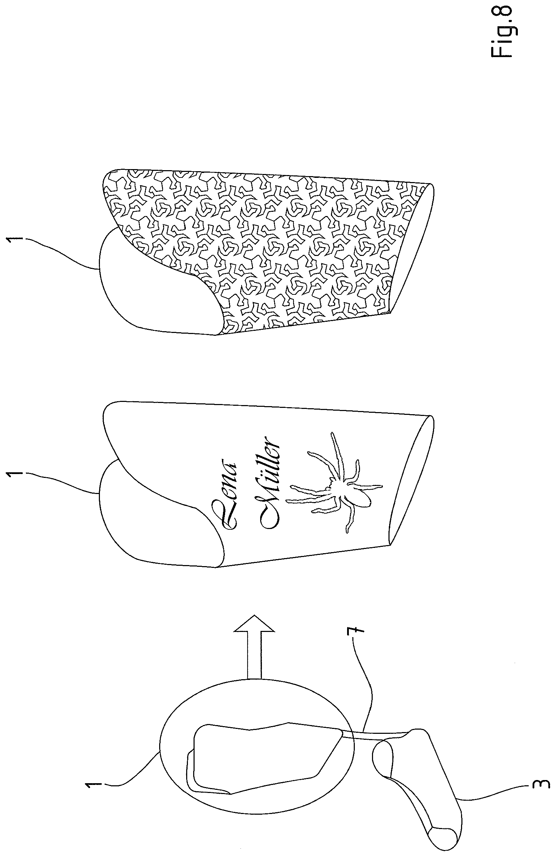

Eine einfachere Variante der "Verschleierung" der medizinisch notwendigen Orthesenform ist in den Fig. 7 und 8 dargestellt. In diesem Fall bleibt die medizinisch notwendige Form insbesondere des Ober- und/oder Unterteils der Orthese im wesentlichen unverändert, wobei hier Einfluss auf die äußere (sichtbare) Oberfläche des Ober- und/oder Unterteils genommen wird. So bietet beispielsweise das "Rapid-Prototyping"-Verfahren die Möglichkeit, beim schichtweisen Aufbau des jeweiligen Orthesenteils Ornamente, Namenszüge oder einfache Oberflächenstrukturierungen auszubilden, welche das äußere Erscheinungsbild des Ober- und/oder Unterteils beeinflussen.

Der Einsatz des "Rapid-Prototyping"-Verfahrens hat jedoch auch andere Vorteile, die nicht indirekt wie die zuvor beschriebene Gestaltungsmöglichkeiten sondern unmittelbar auf den Tragekomfort und damit die Wirksamkeit der Orthese Einfluss nehmen.

Wie eingangs bereits angedeutet wurde, sind Feuchtigkeitsprobleme an der Verschlechterung des Tragekomforts nachhaltig beteiligt. Die Anordnung von atmungsaktiven Polsterungen verkleinern zwar dieses Problem, kreieren jedoch wieder an anderer Stelle Probleme, sodass hierdurch insgesamt keine Vorteile erzielbar sind. Die Anwendung des "Rapid-Prototyping"-Verfahrens ändert dies auf ökonomische Weise.

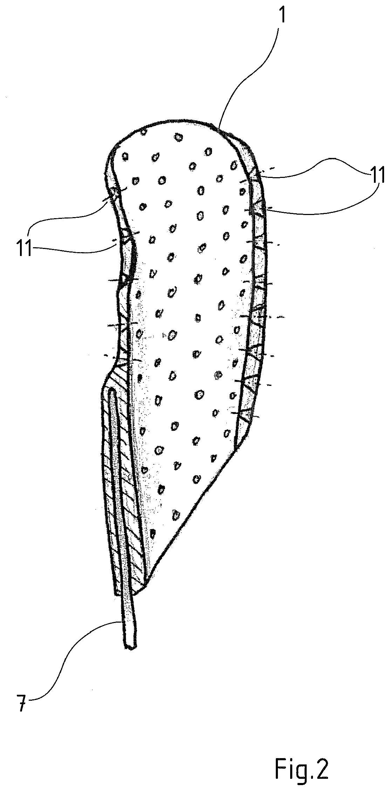

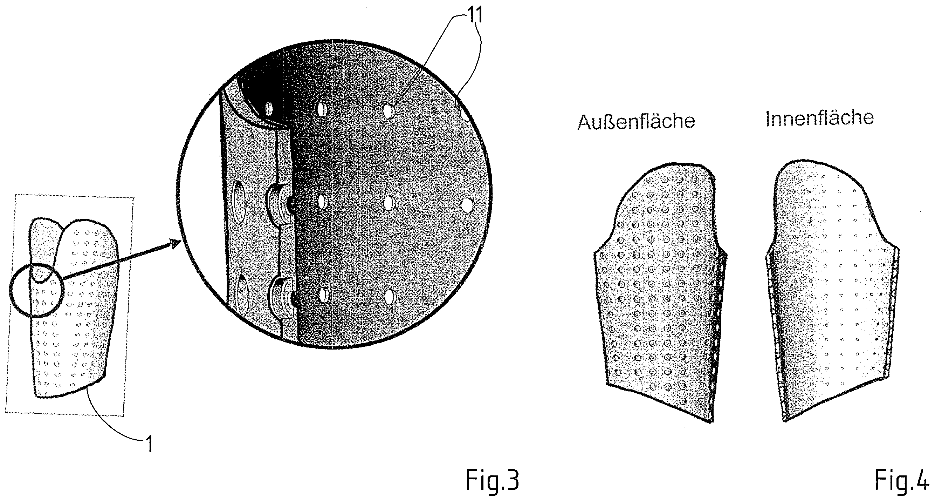

Gemäß der Fig. 2 bis 4 ist es vorgesehen, das Ober-, Mittel- und/oder Unterteil 1-5 einer Orthese mit Perforationsbereichen auszubilden. Vorzugsweise sind das Ober-, Mittel- und/oder Unterteil 1-5 in Form einer Schale ausgebildet, die entweder ein- oder mehrteilig sein kann. Zumindest ein Schalenteil ist zumindest bereichsweise mit einer Anzahl von gleichmäßig oder ungleichmäßig beabstandeten Durchgangslöchern 11 versehen, welche die Schaleninnenseite mit der Schalenaußenseite verbinden. Auf diese Weise kann durch die gezielte Verteilung der Durchgangslöcher 11 ein Muster auf der Schalenoberfläche erzeugt werden, das zur Verschleierung der medizinisch vorgegebenen (natürlichen) Form beiträgt. Vorzugsweise weisen die Durchgangslöcher 11 an der Innenseite einen kleinere Lochdurchmesser auf als an der Außenseite der jeweiligen Schale. Die Durchgangslöcher 11 können dabei kegelförmig (siehen Fig. 2) oder gestuft (siehe Fig. 3 und 4) ausgeformt sein. Sie sind dabei nicht notwendiger Weise kreisförmig, sondern können beliebige Querschnittsformen annehmen (halbkreis-, stern-, sichelförmig, etc.).

An dieser Stelle sei darauf hingewiesen, dass es sich bei den genannten Durchbrüchen 11 nicht um Bohrungen im eigentlichen Sinn handelt, da sie während des Aufbaus des betreffenden Schalenteils nach dem "Rapid-Prototyping"-Verfahren als überlagerte Form ausbildet werden. Daher können die Durchgangslöcher 11 auch einen sich verändernden Querschnitt längs des Durchgangslochs annehmen.

Hierbei sei insbesondere noch auf die Fig. 6 hingewiesen, in der eine konventionelle Orthese solchen Orthesen gegenübergestellt ist, die nach dem erfindungsgemäßen Konzept aufgebaut wurden.

Wie hieraus zu entnehmen ist, erfüllen alle Orthesen die gleiche Aufgabe, nämlich die Stabilisierung eines Sprunggelenks (Fußgelenks) eines Patienten. Die Anwendung des "Rapid-Prototyping"-Verfahrens bietet jedoch die Möglichkeit, die konventionelle Orthese ohne großen Mehraufwand designerisch an den Geschmack des Patienten anzupassen, wobei die Funktionalität und Passgenauigkeit erhalten bleibt. Eine derart angepasste Orthese findet größere Trageakzeptanz und entfaltet so zwangsläufig eine gesteigerte orthopädische Wirkung.

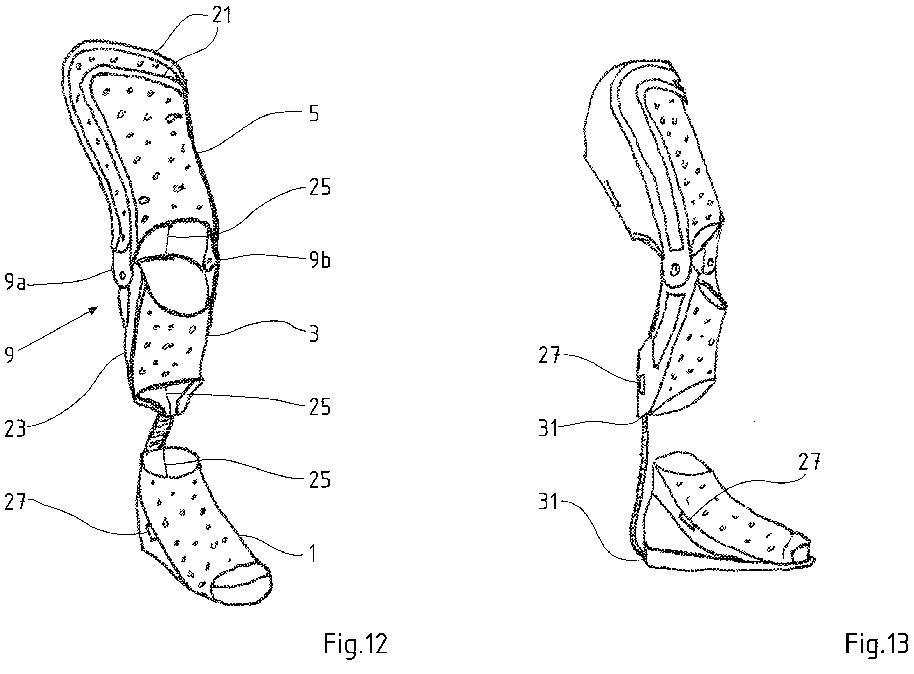

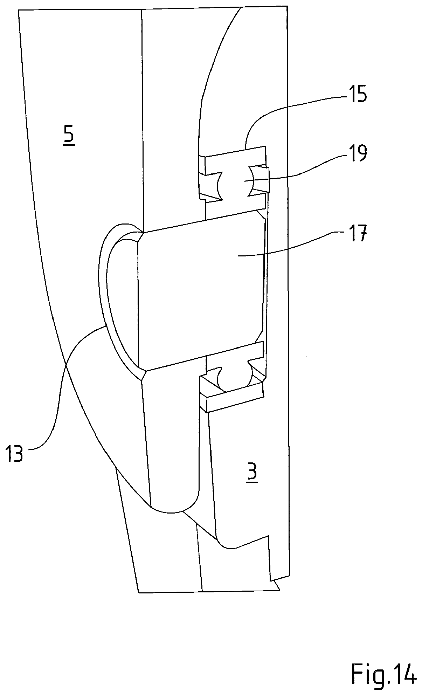

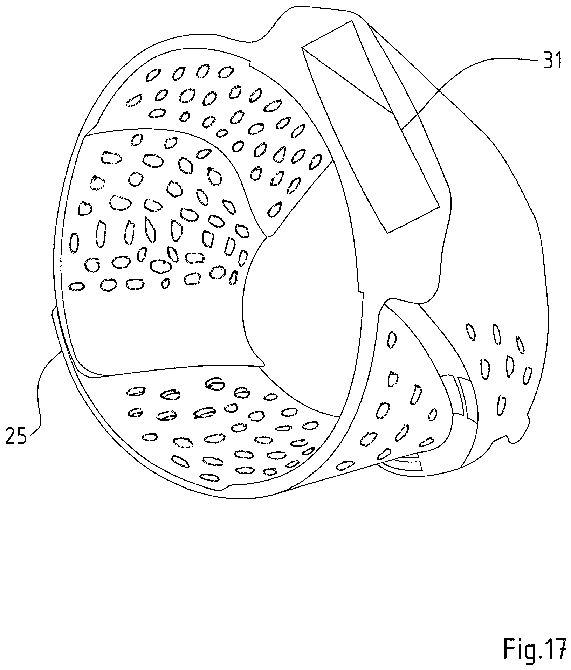

Im Nachfolgenden wird eine konkrete Ausführungsform einer erfindungsgemäßen kombinierten Fuß-Kniegelenk-Orthese anhand der Fig. 12 bis 17 näher beschrieben.

Die erfindungsgemäße Fuß-Kniegelenk-Orthese hat drei seriell angeordnete Körperschalen 1, 3, 5, die eine Oberschenkel- 5 (Oberteil), Unterschenkel- 3 (Mittelteil) und Fußschale 1 (Unterteil) bilden. Die Oberschenkel- und Unterschenkelschale 3, 5 sind über ein Scharniergelenk 9 miteinander gekoppelt, deren Scharnierelemente im Wesentlichen einstückig mit den beiden Schalen 3, 5 ausgebildet sind. Im Konkreten besteht das Scharnier 9 gemäß der Fig. 12 bis 14 aus zwei coaxialen sowie in Breitenrichtung der Schalen beabstandeten Schaniergelenken 9a, 9b, sodass zwischen den Scharniergelenken 9a, 9b das Knie eines Patienten Platz findet. Jedes Scharniergelenk 9a, 9b hat eine Scharnieröse 13, die vorliegend an der Oberschenkelschale 5 ausgebildet ist, sowie eine Lagertasche 15, die vorliegend an der Unterschenkelschale 3 ausgebildet ist, wobei diese beiden Scharnierelemente auch vertauscht sein können. Des Weiteren ist ein Scharnierbolzen 17 durch die Scharnieröse 13 (fest/reibschlüssig) von Außen eingesteckt und endseitig über ein Kugellager 19 in der unterhalb der Scharnieröse 13 liegenden Aufnahmetasche 15 drehbar gelagert.

Zur Stabilisierung von Oberschenkel- und Unterschenkelschale 3, 5 sind an diesen jeweils Versteifungsrippen 21, 23 ausgeformt, die sich vorliegend an der Spannungsverteilung im Schalenmaterial bei deren normaler Benutzung orientieren. Im Konkreten umschließt eine Versteifungsrippe 21 an der Oberschenkelschale 5 eines der Scharnierelemente 9a und erstreckt sich von da in Längsrichtung bis zu deren oberen Schalenrand. Von dort ist die Versteifungsrippe 21 um die Vorderseite des Oberschenkelschale 5 bis zur anderen Seite herum verlängert, um anschließend nach unter bis zum anderen der Scharnierelemente 9b gezogen zu sein. Auf diese Weise wird eine Art Rahmen an der Oberschenkelschale 5 gebildet, der diese verwindungssteifer macht.

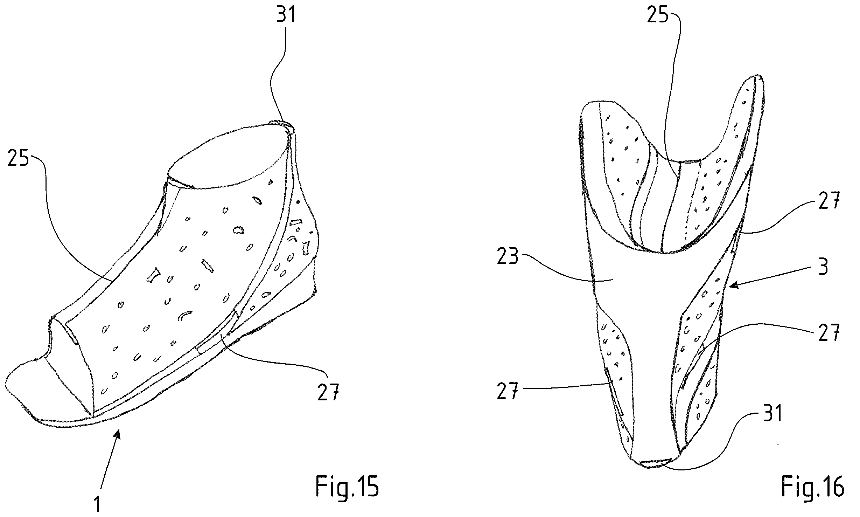

Die Unterschenkelschale 3 weist ebenfalls eine Versteifungsrippe 23 auf, die ausgehend von einem der Scharnierelemente 9a nach unten/hinten gezogen ist, bis diese den unteren Schalenrand erreicht hat, um dann auf der gegenüberliegenden Seite bis zum anderen Scharnierelement 9b wieder hochgezogen zu werden. Hierdurch entsteht von hinten betrachtet (siehe Fig. 16) eine V-Form, deren Schenkel um die Schale herumgeführt sind.

Jeder der drei Schalen 1-5 weist einen durchgehenden Längsschlitz 25 auf, wobei sich die Schalen 1-5 im Schlitzbereich überlappen (siehe Fig. 15, 16). Längs des jeweiligen Schlitzes 25 lassen sich die Schalen 1-5 (elastisch) aufbiegen, sodass sie an einem Körperteil des Patienten angelegt werden können. An allen Schalen 1-5 sind ferner Schlaufen 27 einstückig ausgeformt, in die Gurte oder Schnallen 29 (siehe Fig. 1d, 6) eingesetzt werden können, um die Schalen im Bereich ihrer Längsschlitze 25 verschnüren/verspannen zu können.

Am unteren hinteren Randabschnitt der Unterschenkelschale 3 sowie am oberen, hinteren Randabschnitt der Fußschale 1 sind längs sich erstreckende Aufnahmetaschen 31 einstückig mit den jeweiligen Schalen 1, 3 ausgeformt. Im Querschnitt sind diese Taschen 31 jeweils rechtwinklig, können aber auch eine andere Querschnittsform aufweisen. In die Taschen ist vorliegend ein Federelement 7 in Form einer Blattfeder eingesteckt und darin fixiert. Das Federelement 7 hat dabei eine dem Unterschenkel sowie der Ferse des Patienten nachfolgende Form, um die Relativposition von Unterschenkelschale 3 und Fußschale 1 individuell an die Geometrie eines Patientenbeins anzunähern.

Die Ausgestaltung der kombinierten Knie-Fußgelenk-Orthese erlaubt eine flexible Anwendung nach den individuellen Bedürfnissen des Patienten ohne designerische Änderungen. D.h. Durch Weglassung des Federelements wird die Orthese zu einer reinen Kniegelenkorthese. Durch Weglassung der Oberschenkelschale wird die Orthese zu einer reinen Fußgelenkorthese. Auf diese Weise kann die Trageakzeptanz weiter verbessert und die therapeutische Wirkung der Orthese gesteigert werden.

## Patentansprüche

1. Verfahren zur Herstellung einer Orthese bestehend aus zumindest einem jeweils schalen- oder schellenförmigen Ober- und Unterteil (1-5), die jeweils an die Ergonomie eines zu therapierenden Körperteils eines Patienten angepasst oder angenähert sind, sowie einem das Ober- und Unterteil (1-5) koppelnden Zwischenelement (7, 9), wobei zumindest das Ober- und/oder Unterteil (1-5) der Orthese nach dem "Rapid Prototyping"-Verfahren hergestellt werden, **dadurch gekennzeichnet, dass** die äußere Geometrie des betreffenden Körperteils des Patienten individuell erfasst und als dreidimensionale CAD-Daten digital abgespeichert wird und dass diese derart eingelesenen Ergonomiedaten bezüglich des individuellen Patientenkörperteils mit vorab abgespeicherten standardisierten Formdaten bezüglich eines simulierten Körperteils derart überlagert werden, dass eine Form erhalten wird, die äußerlich dem simulierten Körperteil angenähert ist, deren innere Abmessungen jedoch exakt an die Ergonomie des zu therapierenden Körperteils angepasst ist.

2. Verfahren nach Anspruch 1, **dadurch gekennzeichnet, dass** das Zwischenelement (7, 9) ein Scharniergelenk (9) ist, wobei die zusammenwirkenden Gelenkelemente (9a, 9b) jeweils einstückig mit dem entsprechenden Ober- und Unterteil (3, 5) nach dem "Rapid Prototyping"-Verfahren hergestellt werden.

3. Verfahren nach einem der vorhergehenden Ansprüche, **dadurch gekennzeichnet, dass** durch die Überlagerung mit Standardwerten eine zumindest bereichsweise Perforation zumindest des Ober- und/oder Unterteils (1-5) und/oder eine Strukturierung zumindest der äußeren Oberfläche der Ober- und/oder Unterteils erzeugt wird.

4. Orthese bestehend aus zumindest einem jeweils schalen- oder schellenförmigen Ober- und Unterteil (1-5), die jeweils an die Ergonomie eines zu therapierenden Körperteils eines Patienten angepasst oder angenähert sind, sowie einem das Ober- und Unterteil (1-5) koppelnden Zwischenelement (7, 9), wobei die Orthese nach einem Verfahren gemäß einem der vorhergehenden Patentansprüche hergestellt ist, so dass eine äußere Form durch vorab abgespeicherte standardisierte Formdaten bezüglich eines simulierten Körperteils vorgegeben ist, wobei die inneren Abmessungen exakt an die Ergonomie des zu therapierenden Körperteils angepasst ist.

5. Orthese nach Anspruch 4, wobei das Ober- und/oder Unterteil (1-5) zumindest bereichsweise mit einer Perforation, vorzugsweise Durchgangslöcher (11) zur Belüftung der Teileinnenseite ausgebildet ist, wobei weiter vorzugsweise die Durchgangslöcher (11) an der Teileinnenseite einen kleineren Durchmesser haben als an der Teileaußenseite.

6. Orthese nach Ansprüche 4 oder 5, wobei auf eine sichtbare Außenseite zumindest des Ober- und/oder Unterteils eine Gravur, Prägung oder ein Farbdruck aufgebracht ist.

7. Orthese nach einem der Ansprüche 4 bis 6, wobei zumindest das Ober- und/oder Unterteil (1-5) unter Berücksichtigung individueller geometrischer Abmessungen des zu therapierenden Körperteils eine standardisierte Körperform unterschiedlich zum therapierenden Körperteil simuliert.

8. Orthese nach Ansprüche 4 bis 7, wobei zumindest das Ober- und Unterteil (1-5) mit Versteifungsrippen ausgeformt ist.

## Claims

1. Method for the production of an orthosis consisting of at least one upper and lower part (1-5), each in the form of a shell or clamp, which are adapted or approximated to the ergonomics of a body part of a patient to be treated, and an intermediate element (7, 9) coupling the upper and lower part (1-5), wherein at least the upper and /or lower part (1-5) of the orthosis is produced according to the rapid prototyping method, **characterized in that** the external geometry of the relevant body part of the patient is individually measured and stored digitally as three-dimensional CAD data, and **in that** these ergonomics data read in in such a way with respect to the individual patient body part are superimposed with previously stored standardized shape data with respect to a simulated body part in such a way that a shape is obtained which is externally approximated to the simulated body part, but the internal dimensions of which are exactly adapted to the ergonomics of the body part to be treated.

2. Method according to claim 1, **characterized in that** the intermediate element (7, 9) is a hinge joint (9), wherein the co-operating joint elements (9a, 9b) are each produced integrally with the corresponding upper and lower part (3, 5) using the rapid prototyping method.

3. Method according to one of the preceding claims, **characterized in that** by the superimposition with standard values, a perforation at least in sections of at least the upper and/or lower part (1-5) and/or a structuring of at least the outer surface of the upper and/or lower part is produced.

4. Orthosis consisting of at least one upper and lower part (1-5), each in the form of a shell or clamp, which are adapted or approximated to the ergonomics of a body part of a patient to be treated, and an intermediate element (7, 9) coupling the upper and lower part (1-5), wherein the orthosis is produced according to a method according to one of the preceding patent claims, so that an outer shape is predetermined by previously stored, standardized shape data with respect to a simulated body part, wherein the inner dimensions are exactly adapted to the ergonomics of the body part to be treated.

5. Orthosis according to claim 4, wherein the upper and/or lower part (1-5) is formed at least in sections with a perforation, preferably through-holes (11), for ventilating the inner side of the parts, wherein further preferably the through-holes (11) on the inner side of the parts have a smaller diameter than on the outer side of the parts.

6. Orthosis according to claim 4 or 5, wherein an engraving, embossing or color print is applied to a visible outer side of at least the upper and/or lower part.

7. Orthosis according to one of the claims 4 to 6, wherein at least the upper and/or lower part (1-5) simulates a standardized body shape different from the body part to be treated, taking into account individual geometric dimensions of the body part to be treated.

8. Orthosis according to claims 4 to 7, wherein at least the upper and lower part (1-5) is formed with reinforcing ribs.

## Revendications

1. Procédé de fabrication d'une orthèse constituée d'au moins une partie supérieure et une partie inférieure (1-5) en forme respectivement de coque ou de bride qui sont adaptées respectivement à l'ergonomie d'une partie du corps à soigner d'un patient ou en sont proches, ainsi qu'un élément intermédiaire (7, 9) couplant la partie supérieure et la partie inférieure (1-5), dans lequel au moins la partie supérieure et/ou la partie inférieure (1-5) de l'orthèse sont fabriquées selon le procédé de « prototypage rapide », **caractérisé en ce que** la géométrie extérieure de la partie du corps concernée du patient est saisie individuellement et enregistrée en tant que données CAO tridimensionnelles, et **en ce que** ces données ergonomiques lues de cette façon, relatives à la partie du corps individuelle du patient se superposent aux données de formes normalisées préalablement enregistrées relatives à une partie du corps simulée de manière à obtenir une forme qui soit proche extérieurement de la partie du corps simulée dont les dimensions intérieures sont cependant ajustées exactement à l'ergonomie de la partie du corps à soigner.

2. Procédé selon la revendication 1, **caractérisé en ce que** l'élément intermédiaire (7, 9) est une articulation à charnière (9), dans lequel les éléments d'articulation coopérants (9a, 9b) sont fabriqués respectivement d'un seul tenant avec les parties supérieure et inférieure correspondantes (3, 5) selon le procédé de « prototypage rapide ».

3. Procédé selon l'une quelconque des revendications précédentes, **caractérisé en ce que**, par superposition avec des données normalisées, on obtient une perforation au moins partielle d'au moins la partie supérieure et/ou la partie inférieure (1-5) et/ou une structuration d'au moins la surface extérieure de la partie supérieure et/ou de la partie inférieure.

4. Orthèse constituée d'au moins une partie supérieure et une partie inférieure (1-5) en forme respectivement de coque ou de bride qui sont adaptées respectivement à l'ergonomie d'une partie du corps à soigner d'un patient ou en sont proches, ainsi qu'un élément intermédiaire (7, 9) couplant la partie supérieure et la partie inférieure (1-5), dans laquelle l'orthèse est fabriquée suivant un procédé selon l'une quelconque des revendications précédentes, de sorte qu'une forme extérieure soit prédéterminée par des données de formes normalisées préalablement enregistrées relatives à une partie du corps simulée, dans laquelle les dimensions intérieures sont ajustées exactement à l'ergonomie de la partie du corps à soigner.

5. Orthèse selon la revendication 4, dans laquelle la partie supérieure et/ou inférieure (1-5) est ou sont conçues au moins par endroits avec une perforation, de préférence des trous traversants (11) pour l'aération du côté intérieur des parties, dans laquelle en outre de préférence les trous traversants (11) ont sur le côté intérieur des parties, un diamètre plus petit que sur le côté extérieur des parties.

6. Orthèse selon les revendications 4 ou 5, dans laquelle on applique sur un côté extérieur visible d'au moins la partie supérieure et/ou inférieure une gravure, un gaufrage ou une impression couleur.

7. Orthèse selon l'une quelconque des revendications 4 à 6, dans laquelle au moins la partie supérieure et/ou la partie inférieure (1-5) stimule(nt) en prenant en compte les dimensions géométriques individuelles de la partie du corps à soigner, une forme corporelle normalisée différemment de la partie du corps à soigner.

8. Orthèse selon les revendications 4 à 7, dans laquelle au moins la partie supérieure et la partie inférieure (1-5) sont formées avec des nervures de renfort.
